(19) Europäisches Patentamt
European Patent Office
Office européen des brevets

(11) **EP 3 323 373 A2**

(12) **EUROPÄISCHE PATENTANMELDUNG**

(43) Veröffentlichungstag:
**23.05.2018 Patentblatt 2018/21**

(51) Int Cl.:
***A61B 90/00*** (2016.01)   ***A61B 17/16*** (2006.01)
***A61B 34/20*** (2016.01)   *A61B 17/00* (2006.01)

(21) Anmeldenummer: **17201010.0**

(22) Anmeldetag: **10.11.2017**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**MA MD**

(30) Priorität: **17.11.2016 DE 102016222645**

(71) Anmelder: **Airbus Defence and Space GmbH
82024 Taufkirchen (DE)**

(72) Erfinder: **BOSE, Guido
88090 Immenstaad (DE)**

(74) Vertreter: **Frenkel, Matthias Alexander
Wuesthoff & Wuesthoff
Patentanwälte PartG mbB
Schweigerstrasse 2
81541 München (DE)**

(54) **VERFAHREN ZUM STEUERN EINES MEDIZINISCHEN BOHRSYSTEMS UND EIN MEDIZINISCHES BOHRSYSTEM**

(57)   Es wird ein Verfahren zum Steuern eines medizinischen Bohrsystems bereitgestellt. Das Verfahren umfasst kontinuierliches Aussenden von Meßstrahlen. Das Verfahren umfasst ferner kontinuierliches Empfangen der teilweise an verschiedenen Gewebearten reflektierten Meßstrahlen. Das Verfahren umfasst ferner Verarbeiten der kontinuierlich empfangenen Meßstrahlen zu Abstandsinformationen. Die Abstandsinformationen geben Abstände zwischen einem Bohrkopf und den verschiedenen Gewebearten an. Das Verfahren umfasst ferner Auswerten, basierend auf den Abstandsinformationen, ob es erlaubt ist, in Richtung einer Bohrung des Bohrkopfs zu bohren. Das Verfahren umfasst ferner Steuern des Bohrkopfs, basierend auf dem Auswerten. Ferner wird medizinisches Bohrsystem bereitgestellt.

Fig.2

EP 3 323 373 A2

**Beschreibung**

Die Erfindung betrifft ein Verfahren zum Steuern eines medizinischen Bohrsystems und ein medizinisches Bohrsystem.

In der Human- und Veterinärmedizin kann es bei einem operativen Eingriff bei einem Patienten zu Verletzungen kommen, wenn Knochenteile des Patienten gebohrt werden müssen. Diese Verletzungen können durch Schädigungen von zum Beispiel Nerven entstehen. Bisher hat man während eines medizinischen Bohrvorgangs beim Patienten keinerlei Indikation, wie weit der Bohrer von einer Nervenbahn bzw. sensitiven Bereichen entfernt ist. Hierbei besteht die Gefahr besonders bei sehr sensiblen Operationen, wie zum Beispiel Hirnoperationen, dass Nervenbahnen oder anderes empfindliches Gewebe irreparabel beschädigt werden können. Dies kann zusätzliche Folgeschädigungen nach sich ziehen.

Es ist nun Aufgabe der vorliegenden Erfindung effiziente Operationen ohne Nervenschädigungen, Wirbelschädigung, Knochenschädigung oder Hirnschädigung bei einem Patienten während eines operativen Eingriffs in der Human oder Veterinärmedizin zu vermeiden.

Ein erster Aspekt betrifft ein Verfahren zum Steuern eines medizinischen Bohrsystems. Das Verfahren umfasst kontinuierliches Aussenden von Meßstrahlen. Das Verfahren umfasst ferner kontinuierliches Empfangen der teilweise an verschiedenen Gewebearten reflektierten Meßstrahlen. Die teilweise an den verschiedenen Gewebearten reflektierten Meßstrahlen können den ausgesendeten Meßstrahlen entsprechen, die an verschiedenem Gewebe zurückreflektiert werden. Die Meßstrahlen können durch den Bohrkopf hindurch ausgesendet und empfangen werden. Dabei können die reflektierten Meßstrahlen mindestens in ihrer Amplitude aufgrund von Dämpfung und Reflexion vermindert sein. Das Verfahren umfasst ferner Verarbeiten der kontinuierlich empfangenen Meßstrahlen zu Abstandsinformationen. Die Abstandinformationen geben Abstände zwischen einem Bohrkopf und den verschiedenen Gewebearten an. Das Verfahren umfasst ferner Auswerten, basierend auf den Abstandsinformationen, ob es erlaubt ist, in Richtung einer Bohrung des Bohrkopfs zu bohren. Das Verfahren umfasst ferner Steuern des Bohrkopfs, basierend auf dem Auswerten.

Dies hat den Vorteil, Verletzungen bei einem Patienten während eines operativen Eingriffs zu vermeiden. Dies hat zusätzlich den Vorteil, Bestrahlung durch Röntgen auf ein Minimum zu reduzieren.

Hierbei kann ein Abstand zwischen einem Bohrkopf und den verschiedenen Gewebearten dadurch bestimmt sein, dass eine Sensoreinheit, die in dem Bohrkopf angebracht sein kann, den Ursprung der Messung bildet. Ferner kann der Abstand auch durch Herausrechnen einer Entfernung zwischen dem vordersten Ende des Bohrkopfes und der Sensoreinheit konkretisiert werden.

Das Verfahren kann ferner Anzeigen, basierend auf dem Auswerten, von zu bohrenden und nicht zu bohrenden Gewebearten umfassen. Zu bohrende Gewebearten können Knochenteile und Knochenstrukturen sein, die zur Anbringung von Schrauben gebohrt werden sollen oder selbst zur Verbindung mit anderen Knochenteilen verbunden werden sollen. Nicht zu bohrende Gewebearten können Weichteile und insbesondere Nerven sein, die zu irreparablen Schädigungen bei einem Patienten führen können.

Dies hat den Vorteil, einem Operateur anzuzeigen, ob das zu bohrende Gewebe oder das in Zukunft zu bohrende Gewebe nicht gebohrt werden soll, womit der Operateur die Möglichkeit hat im Sinne des Patienten zu reagieren.

Das Anzeigen kann ferner Anzeigen von unterschiedlichen Gefahrenstufen der nicht zu bohrenden Gewebearten umfassend. Das Anzeigen kann auch Anzeigen von Vorwarnungen für in Richtung der Bohrung liegende nicht zu bohrende Gewebearten umfassen. Das Anzeigen kann auch Anzeigen von Vorwarnungen für in angrenzender Nähe der Bohrung liegende nicht zu bohrende Gewebearten umfassen. In angrenzender Nähe der Bohrung liegende nicht zu bohrende Gewebearten können Hohlräume sein, die zu einem Brechen eines zu bohrenden Knochenteils führen können, falls zu nahe daran gebohrt wird.

Hiermit kann erreicht werden, dass dem Operateur mitgeteilt werden kann, ob und wie weit entsprechendes Gewebe von dem Bohrkopf entfernt liegt, und ob eine Operation weiter sinnvoll durchgeführt werden kann.

Das Verfahren kann ferner Auslösen eines Signals umfassen, wenn das Auswerten ergibt, dass es nicht erlaubt ist, mindestens eine der verschiedenen Gewebearten in Richtung der Bohrung zu bohren. Das ausgelöste Signal kann ein akustisches Signal über eine Lautsprechereinheit an einem mit dem Bohrer verbundenen System sein. Das ausgelöste Signal kann ein Vibrationssignal sein, das über den Bohrer eine Vibration abgibt, um einem Operateur eine haptisches Signal zu geben, dass er nicht weiter bohren soll.

Dieser den Vorteil, dem Operateur direkt mitzuteilen, ob zu beschädigendes Gewebe vorliegt, worauf der Operateur unmittelbar reagieren kann.

Das Steuern des Bohrkopfs kann ferner Stoppen der Bohrung vor Eindringen des Bohrkopfs in mindestens eine der nicht zu bohrenden Gewebearten umfassen. Hierbei kann der Bohrkopf mit einer Abschaltautomatik versehen sein, die über ein Ampelsystem anzeigt, zum Beispiel eine Ampel anzeigend Rot für gefährlich, gelb für mittelgefährlich und grün für kein Problem, ob der Bohrer sich in Richtung der Bohrung einer nicht zu bohrenden Gewebeart nähert.

Dies hat ferner den Vorteil, dass automatisch der Bohrprozess abgeschaltet werden kann.

Die Abstandsinformationen können basierend auf einem Dämpfungsgesetz berechnet werden. Es kann sich je nach Untersuchungsgebiet eine unterschiedliche Dämpfung durch unterschiedliche Absorptionskoeffizienten und frequenzabhängiger Eindringtiefe ergeben. Das Untersuchungsgebiet kann sich durch die Ausrichtung der Bohrung ergeben. Es können charakteristische Frequenzen für zu bohrende Gewebearten und nicht zu bohrende Gewebearten für das Berechnen verwendet werden. Ferner kann sich daraus eine unterschiedliche Geschwindigkeit in unterschiedlichen Medien, hier den Gewebearten, ergeben. Zusätzlich können akustische Impedanzen für die Reflexion an Grenzflächen zwischen unterschiedlichen Gewebearten entscheidend für die Reflexion der ausgesendeten Meßstrahlen sein. Somit können unterschiedliche Laufzeiten zwischen zu bohrenden und nicht zu bohrenden Gewebearten entstehen. Die so ermittelte Laufzeitmessung kann in den Abstandsinformationen enthalten sein.

Diese zusätzlichen Merkmale haben den Vorteil, dass entsprechend genau eine Laufzeit von von dem Sensor ausgesendeten Meßstrahlen berechnet werden kann. Damit kann einem Operateur genaueres Feedback gegeben werden. Der Operateur kann dadurch schneller und effizienter auf sein Vorhaben reagieren.

Die einzelnen Schritte des Verfahrens können während eines Betriebs des Bohrers ausgeführt werden.

Ein zweiter Aspekt betrifft ein Computerprogramm zur Durchführung eines Verfahrens nach dem ersten Aspekt.

Ein dritter Aspekt betrifft ein Computerprogrammprodukt beinhaltend einen maschinenlesbaren Programmträger, auf dem ein Computer-Programm gemäß dem zweiten Aspekt in der Form von elektronisch und/oder optisch lesbaren Steuersignalen gespeichert ist.

Ein vierter Aspekt betrifft ein medizinisches Bohrsystem. Das medizinische Bohrsystem umfasst einen Bohrer, eine Sensoreinheit, eine Verarbeitungseinheit und eine Steuereinheit. Der Bohrer ist konfiguriert, verschiedene Gewebearten zu bohren. Der Bohrer umfasst einen Bohrkopf.

Die Sensoreinheit ist konfiguriert, Meßstrahlen kontinuierlich auszusenden. Die Sensoreinheit ist ferner konfiguriert, die teilweise an verschiedenen Gewebearten reflektierten Meßstrahlen kontinuierlich zu empfangen. Die Verarbeitungseinheit ist konfiguriert, die kontinuierlich empfangenen Meßstrahlen zu Abstandsinformationen zu verarbeiten. Die Abstandsinformationen umfassen Informationen über Abstände von dem Bohrkopf zu den verschiedenen Gewebearten. Die Verarbeitungseinheit ist ferner konfiguriert, basierend auf den Abstandsinformationen auszuwerten, ob es erlaubt ist, in Richtung einer Bohrung des Bohrkopfs zu bohren. Die Steuereinheit ist konfiguriert, den Bohrkopf, basierend auf dem Auswerten zu steuern.

Die Sensoreinheit kann sich zumindest teilweise in dem Bohrkopf befinden. Die Sensoreinheit kann eine Abstrahlcharakteristik aufweisen, die in Richtung einer Bohrung weist. Zusätzlich kann die Sensoreinheit eine Abstrahlcharakteristik aufweisen, die in Richtung senkrecht zur Bohrung weist. Hierfür kann die Sensoreinheit zwei senkrecht zueinander ausgerichtete Sensoren aufweisen. Ferner kann eine Abstrahlcharakteristik einer Empfangscharakteristik entsprechen.

Die Anbringung der Sensoreinheit kann dadurch vorteilhaft sein, dass etwaige Dämpfungen verringert werden können.

Die Sensoreinheit kann aus einem oder mehreren Sensoren, vorzugsweise Ultraschallsensoren, bestehen. Dabei können mindestens ein Sensor zum Senden und mindestens ein Sensor zum Empfangen bereitgestellt sein. Der mindestens eine Sensor zum Senden und der mindestens eine Sensor zum Empfangen, können auch die Sensoreinheit bilden. Diese können auch respektive als Sender und Empfänger bezeichnet sein.

Die Sensoreinheit kann ferner einen Ultraschallsensor umfassen. Der Ultraschallsensor kann dazu ausgebildet sein Meßstrahlen zu senden. Ferner kann der Ultraschallsensor dazu ausgebildet sein, Echos der gesendeten Meßstrahlen zu empfangen. Die Verarbeitungseinheit kann ausgebildet sein, die Abstandsinformationen durch Laufzeitmessungen basierend auf einer Berechnung von akustischen Impedanzen zu berechnen. Die akustische Impedanz kann sich durch die Multiplikation von Schallgeschwindigkeit und Dichte der jeweiligen Gewebeart ergeben. Die Laufzeit kann sich durch ausgesendete Meßstrahlen mit den dazugehörigen Echos ergeben. Ferner kann die Verarbeitungseinheit dazu ausgebildet sein, basierend auf den Laufzeitmessungen, Abstandinformationen zu berechnen. Ferner kann die Verarbeitungseinheit ausgebildet sein, basierend auf den Abstandinformationen, ein Bild zu erstellen. Hierzu kann die Verarbeitungseinheit dazu ausgebildet sein, ein Reflexionen Diagramm zu berechnen. Basierend auf dem Reflexionen Diagramm kann die Verarbeitungseinheit ausgebildet sein, ein Signal auszulösen oder den Bohrprozess zu stoppen.

Ferner kann die Sensoreinheit ein optisches Interferometer sein. Das optische Interferonmeter kann ausgebildet sein, Meßstrahlen zu senden. Das Prinzip kann auf der optischen Kohärenztomographie beruhen. Dabei kann Licht geringer Kohärenzlänge mithilfe eines Interferometers zur Entfernungsmessung streuender Gewebearten eingesetzt werden. Dazu kann ein zu untersuchendes Objekt punktweise abgetastet werden.

Die einzelnen Einheiten des medizinischen Bohrsystems können so vorgesehen sein, dass sie während eines Betriebs des Bohrers eingeschaltet/aktiv sind.

Es ist dem Fachmann klar, dass die hierin dargelegten Erklärungen unter Verwendung von Hardwareschaltungen, Softwaremitteln oder einer Kombination davon implementiert sein/werden können. Die Softwaremittel können im Zusammenhang stehen mit programmierten Mikroprozessoren oder einem allgemeinen Computer, einer ASIC (Application Specific Integrated Circuit; zu Deutsch: anwendungsspezifische integrierte Schaltung) und/oder DSPs (Digital Signal Processors;

zu Deutsch: digitale Signalprozessoren). Beispielsweise kann die genannte Verarbeitungseinheit teilweise als ein Computer oder ein Prozessor (beispielsweise einen Mikroprozessor, Mikrocontroller oder DSP) realisiert sein. Es ist dem Fachmann zu dem klar, dass auch dann wenn die hierin beschriebenen Details in Bezug auf ein Verfahren beschrieben werden, diese Details auch in einer geeigneten Vorrichtungseinheit, einem Computerprozessor oder einem mit einem Prozessor verbundenen Speicher realisiert sein können, wobei der Speicher mit einem oder mehreren Programmen versehen ist, die das Verfahren durchführen, wenn sie durch den Prozessor ausgeführt werden.

Auch wenn einige der voranstehend beschriebenen Aspekte in Bezug auf das Verfahren beschrieben wurden, so können diese Aspekte auch auf das medizinische Bohrsystem zutreffen. Genauso können die voranstehend in Bezug auf das medizinische Bohrsystem beschriebenen Aspekte in entsprechender Weise auf das Verfahren zutreffen.

Weitere Ziele, Merkmale, Vorteile und Anwendungsmöglichkeiten ergeben sich aus der nachfolgenden Beschreibung von nicht einschränkend zu verstehenden Ausführungsbeispielen mit Bezug auf die zugehörigen Zeichnungen. Dabei zeigen alle beschriebenen und/oder bildlich dargestellten Merkmale für sich oder in beliebiger Kombination den hier offenbarten Gegenstand, auch unabhängig von ihrer Gruppierung in den Ansprüchen oder deren Rückbeziehungen. Die Abmessungen und Proportionen der in den Figuren gezeigten Komponenten sind hierbei nicht unbedingt maßstäblich; sie können bei zu implementierenden Ausführungsformen vom hier Veranschaulichten abweichen.

Figur 1　　　schematische Darstellung eines Prinzipschaltbilds eines medizinischen Bohrsystems;

Figur 2　　　schematische Darstellung eines Verfahrens zum Steuern eines medizinischen Bohrsystems;

Figur 3A　　schematische Darstellung des Dämpfungsgesetzes mit Abhängigkeiten;

Figur 3B　　schematische Darstellung eines Diagramms mit frequenzmäßiger Abhängigkeit eines Untersuchungsgebiets/Eindringtiefe;

Figur 3C　　schematische Darstellung eines Diagramms mit Laufzeitabhängigkeiten von verschiedenen Gewebearten;

Figur 3D　　schematische Darstellung eines Diagramms mit akustischen Impedanzen;

Figur 3E　　schematische Darstellung eines Diagramms mit Gewebearten und dazugehörigen Dämpfungswerten;

Figur 4A　　schematische Darstellung eines Bohrers mit Bohrer, Bohrkopf, und Sensoreinheit;

Figur 4B　　schematische Darstellung eines Bohrkopfs und Meßstrahlen;

Figur 5A　　schematische Darstellung eines Reflexionen Diagramms anzeigend verschiedene Laufzeitmessungen; und

Figur 5B　　schematische Darstellung eines Puls-Doppler Verfahrens.

Die hier beschriebenen Verfahrensvarianten der sowie deren Funktions- und Betriebsaspekte dienen lediglich dem besseren Verständnis ihrer Struktur, Funktionsweise und Eigenschaften; sie schränken die Offenbarung nicht etwa auf die Ausführungsbeispiele ein. Die Figuren sind teilweise schematisch, wobei wesentliche Eigenschaften und Effekte zum Teil deutlich vergrößert dargestellt sind, um die Funktionen, Wirkprinzipien, technischen Ausgestaltungen und Merkmale zu verdeutlichen. Dabei kann jede Funktionsweise, jedes Prinzip, jede technische Ausgestaltung und jedes Merkmal, welches/welche in den Figuren oder im Text offenbart ist/sind, mit allen Ansprüchen, jedem Merkmal im Text und in den anderen Figuren, anderen Funktionsweisen, Prinzipien, technischen Ausgestaltungen und Merkmalen, die in dieser Offenbarung enthalten sind oder sich daraus ergeben, frei und beliebig kombiniert werden, so dass alle denkbaren Kombinationen den beschriebenen Vorrichtungen zuzuordnen sind. Dabei sind auch Kombinationen zwischen allen einzelnen Ausführungen im Text, das heißt in jedem Abschnitt der Beschreibung, in den Ansprüchen und auch Kombinationen zwischen verschiedenen Varianten im Text, in den Ansprüchen und in den Figuren umfasst und können zum Gegenstand weiterer Ansprüche gemacht werden. Auch die Ansprüche limitieren nicht die Offenbarung und damit die Kombinationsmöglichkeiten aller aufgezeigten Merkmale untereinander. Alle offenbarten Merkmale sind explizit auch einzeln und in Kombination mit allen anderen Merkmalen hier offenbart.

In den Figuren sind einander entsprechende oder funktionsähnliche Bauteile mit gleichen Bezugszeichen versehen. Das erfindungsgemäße Verfahren und das erfindungsgemäße medizinische Bohrsystem werden nun anhand von Ausführungsbeispielen beschrieben.

Im Folgenden werden ohne hierauf beschränkt zu sein, spezifische Details dargelegt, um ein vollständiges Verständnis der vorliegenden Offenbarung zu liefern. Es ist einem Fachmann jedoch klar, dass die vorliegende Offenbarung in anderen Ausführungsbeispielen verwendet werden kann, die von den nachfolgend dargelegten Details abweichen können.

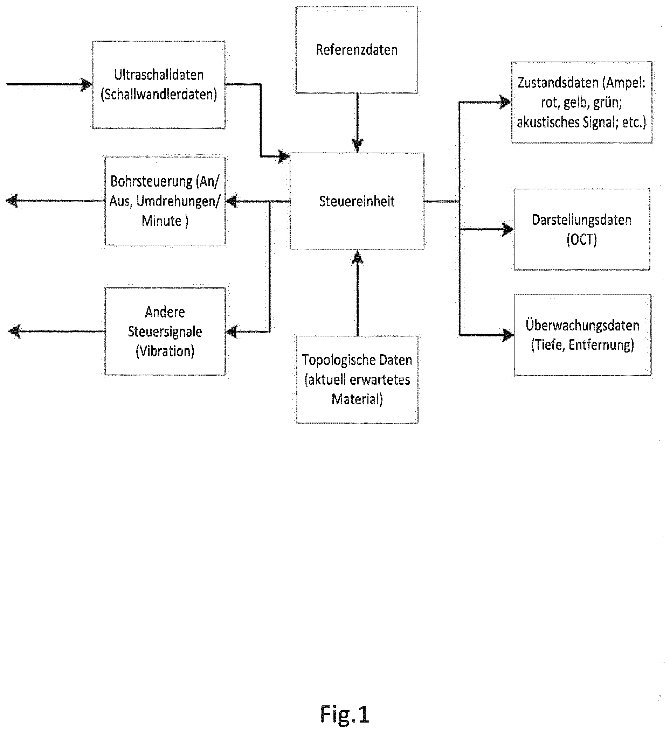

Figur 1 zeigt eine schematische Darstellung eines Prinzipschaltbilds eines medizinischen Bohrsystems. Das medizinische Bohrsystem umfasst einen Bohrer, eine Sensoreinheit, eine Verarbeitungseinheit und eine Steuereinheit. In Figur 1 ist die Verarbeitungseinheit und die Sensoreinheit in die Steuereinheit integriert dargestellt. Die Steuereinheit kann ferner durch eine Software implementiert sein, die zum Beispiel durch einen Prozessor, ausgeführt wird. Der Bohrer ist konfiguriert, verschiedene Gewebearten zu bohren.

Die Sensoreinheit ist konfiguriert, Meßstrahlen kontinuierlich auszusenden. Die Sensoreinheit ist ferner konfiguriert, die teilweise an verschiedenen Gewebearten reflektierten Meßstrahlen kontinuierlich zu empfangen. Die Verarbeitungseinheit ist konfiguriert, die kontinuierlich empfangenen Meßstrahlen zu Abstandsinformationen zu verarbeiten. Die Abstandsinformationen umfassen Informationen über Abstände von dem Bohrkopf zu den verschiedenen Gewebearten. Die Verarbeitungseinheit ist ferner konfiguriert, basierend auf den Abstandsinformationen auszuwerten, ob es erlaubt ist, in Richtung einer Bohrung des Bohrkopfs zu bohren. Die Steuereinheit ist konfiguriert, den Bohrkopf, basierend auf dem Auswerten zu steuern. Der Bohrer umfasst einen Bohrkopf. Dieser Bohrkopf wird über eine Bohrsteuerung, zum Beispiel an/aus, oder durch Einstellen der Umdrehungen pro Minute, durch die Steuereinheit, gesteuert. Ferner können über die Steuereinheit andere Steuersignale, wie zum Beispiel Vibrationen über den Bohrer, übertragen werden. Ferner können Ultraschalldaten (Schallwandlerdaten) von der Sensoreinheit empfangen werden. Die empfangenen Ultraschalldaten können durch die Verwaltungseinheit verarbeitet werden, wodurch die Steuereinheit dazu befähigt wird, eine Aktion auszuführen. Ferner kann eine Anzeigeeinheit vorgesehen sein, die einem Operateur Informationen über zu bohrende Gewebearten anzeigt. Hierfür werden Referenzdaten und topologische Daten verwendet. Topologische Daten zeigen ein aktuell erwartetes Material bezüglich einer bestimmten Gewebeart an. Ferner können die Referenzdaten und topologischen Daten für die Berechnung der Laufzeitunterschiede verwendet werden. Die Laufzeitunterschiede von verschiedenen Meßstrahlen können für einen Vergleich der Abstandsinformation verwendet werden. Hierfür kann zum Beispiel ein Dopplerverfahren angewandt werden. Das Dopplerverfahren beruht auf dem Doppler Effekt. Dabei kommt es zu einer Veränderung der Frequenz, wenn sich der Reflexionspunkt nähert oder entfernt. Ferner kann hierdurch eine Differenzfrequenz berechnet werden, die sich durch Interferenz der ausgesendeten Meßstrahlen und der dazugehörigen Echos ergeben. Voraussetzung dafür ist eine entsprechende Bewegung, die zu einer Frequenzveränderung führt.

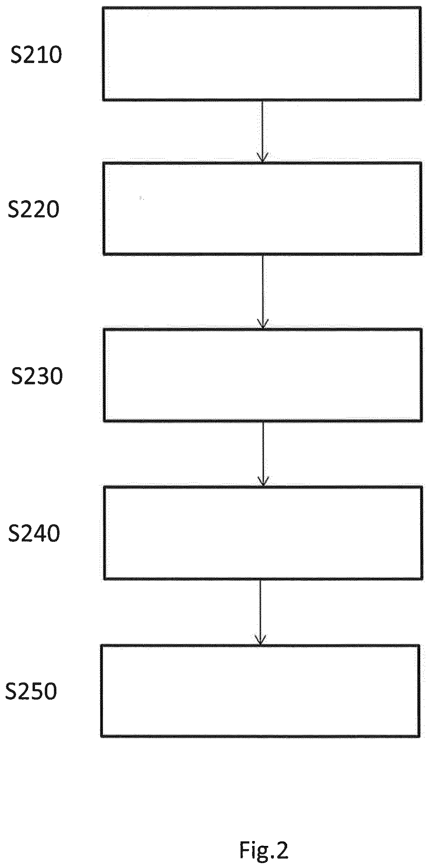

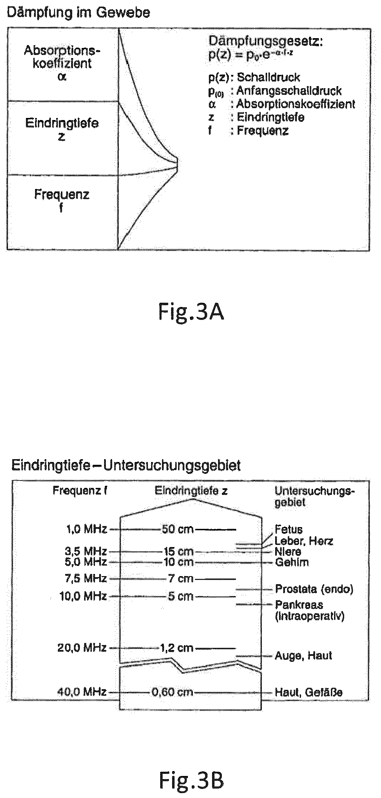

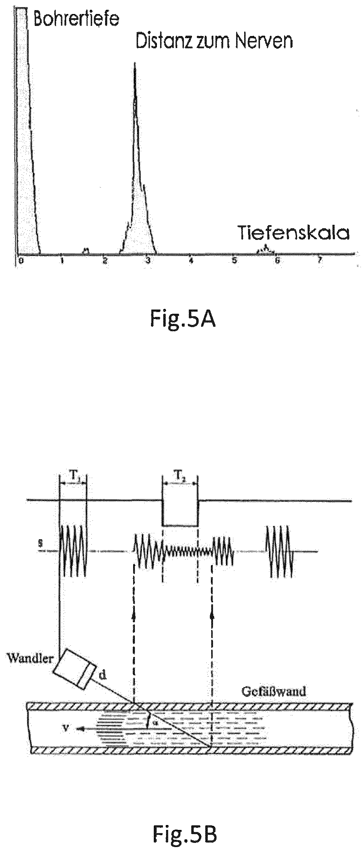

Figur 2 zeigt eine schematische Darstellung eines Verfahrens zum Steuern eines medizinischen Bohrsystems. Das Verfahren umfasst kontinuierliches Aussenden S210 von Meßstrahlen. Das Verfahren umfasst ferner kontinuierliches Empfangen S220 von teilweise an verschiedenen Gewebearten reflektierten Meßstrahlen. Die teilweise an den verschiedenen Gewebearten reflektierten Meßstrahlen können den ausgesendeten Meßstrahlen entsprechen, die an verschiedenem Gewebe zurückreflektiert werden. Dabei können die empfangenen Meßstrahlen aufgrund von Dämpfung und Reflexion von den ausgesendeten Meßstrahlen verringert sein. Die empfangenen Meßstrahlen können auch als Echos der ausgesendeten Meßstrahlen bezeichnet werden. Das Verfahren umfasst ferner Verarbeiten S230 der kontinuierlich empfangenen Meßstrahlen zu Abstandsinformationen. Die Abstandinformationen geben Abstände zwischen einem Bohrkopf und den verschiedenen Gewebearten an. Das Verfahren umfasst ferner Auswerten S240, basierend auf den Abstandsinformationen, ob es erlaubt ist, in Richtung einer Bohrung des Bohrkopfs zu bohren. Das Verfahren umfasst ferner Steuern S250 des Bohrkopfs, basierend auf dem Auswerten. Der Schritt des Auswertens kann ferner ein Anwenden eines Puls-Doppler Verfahrens umfassen wie in Figur 5B dargestellt. Weitere mögliche Verarbeitungsschritte werden in den folgenden Figuren 3A bis 3E und Figur 5A und 5B dargestellt.

Figur 3A zeigt eine schematische Darstellung des Dämpfungsgesetzes mit Abhängigkeiten. Die Sensoreinheit umfassend einen oder mehrere Sensoren zum empfangen und senden entsprechend Figur 1 kann dazu ausgebildet sein, Schall auszusenden (hier der Meßstrahl), der beim Übergang zwischen Medien verschiedene Dichte seine Schallgeschwindigkeit ändert. An Übergangsflächen verschiedener Gewebearten kommt es zu Reflexion des ausgesendeten Schalls. Das wird dazu verwendet, ein Ultraschallbild in der Medizin zu erzeugen. Entsprechende Software für ein solches Ultraschallsystem ermöglicht es, schnell Informationen über die Struktur einer Gewebeart zu erlangen, ohne dass es dabei zu einer Schädigung des entsprechenden Körperteils respektive Gewebes kommt. Hierdurch wird grundsätzlich der Patient durch weniger Röntgenuntersuchungen entlastet. Zur Laufzeitmessung kann ein Zeitunterschied zwischen einem ausgesendeten Meßstrahl und dem dazugehörigen Echo unter Verwendung einer Schallgeschwindigkeit in der spezifischen Gewebeart berechnet werden. Hierzu kann eine Dämpfung in der spezifischen Gewebeart über das Dämpfungsgesetz berechnet werden. Das Dämpfungsgesetz ergibt sich aus

$$p(z) = p_0 \cdot e^{-\alpha \cdot f \cdot z},$$

wobei $\alpha$ der Absorptionskoeffizient, z die Eindringtiefe, f die Frequenz, $p_0$ der Anfangsschalldruck und p(z) der Schalldruck ist.

Figur 3B zeigt eine schematische Darstellung eines Diagramms mit frequenzmäßiger Abhängigkeit eines Untersuchungsgebiets/Eindringtiefe. Das Wissen aus dieser schematischen Darstellung kann verwenden werden, um für verschiedene Eindringtiefe an verschiedenen Gewebearten eine nötige Schallfrequenz einzustellen. Dazu kann die Sensoreinheit dazu ausgebildet sein, entsprechend einer zu untersuchenden vorbestimmten Gewebeart eine Frequenz oder einen Frequenzbereich einzustellen, der auf die zu untersuchende Gewebeart eingestellt ist.

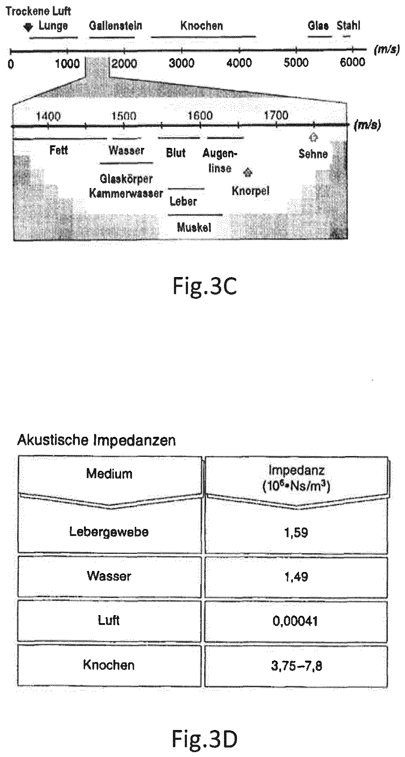

Figur 3C zeigt eine schematische Darstellung eines Diagramms mit Laufzeitabhängigkeiten von verschiedenen Gewebearten. Hier wird abhängig von dem jeweiligen Material/der jeweiligen Gewebeart eine entsprechende Geschwindigkeit eines Schalls dargestellt. Basierend auf vorbestimmten Werten für Laufzeiten in verschiedenen Gewebearten, können bei einer Laufzeitberechnung zu bohrende und nicht zu bohrende Gewebearten unterschieden werden.

Figur 3D zeigt eine schematische Darstellung eines Diagramms mit akustischen Impedanzen. Die akustischen Impedanzen werden hier für ein jeweiliges Medium dargestellt, wobei Knochen die Höchstimpedanz darstellt, wodurch hier am meisten Reflexionen auftreten.

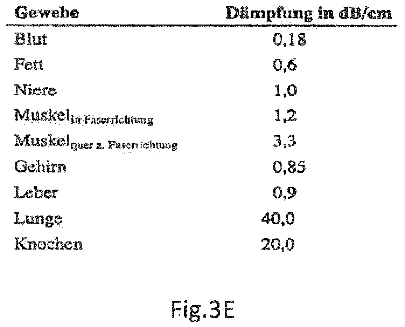

Figur 3E zeigt eine schematische Darstellung eines Diagramms mit Gewebearten und dazugehörigen Dämpfungswerten. Genauso wie die in den Figuren 3A bis 3-D gezeigten schematischen Darstellung, kann eine Dämpfung gemäß der Figur 3E für die Unterscheidung verschiedener Gewebearten verwendet werden, um dadurch eine Laufzeit zu berechnen. Ferner kann hierauf die Berechnung des Laufzeitunterschieds zwischen zu bohrenden und nicht zu bohrenden Gewebearten beruhen.

Die Figuren 3A bis 3E spielen derart zusammen, dass hieraus Impedanzunterschiede den verschiedenen Gewebearten zugeordnet werden können. Somit können Abstandsinformationen zu verschiedenen Gewebearten bestimmt werden. Ferner kann eine Berechnung über eine Verwendung des Dämpfungsgesetzes ausgeführt werden. Alle möglichen Organe und Flüssigkeiten im Körper können im Rahmen des Dämpfungsgesetzes zur Identifikation der Unterschiedlichkeiten der verschiedenen Gewebearten vorgegeben sein. Diese Vorgaben ermöglichen eine Zuordnung der verschiedenen Gewebearten. Über eine Eindringtiefe der Meßstrahlen kann auf ein bestimmtes Gewebe geschlossen werden. Hierzu können Laufzeitunterschiede in Verbindung mit einem Amplitudenvergleich zur Bestimmung der Abstandsinformationen verwendet werden. Für die Abstandsinformationen können ferner Referenzwerte, zum Beispiel aus einer Lookup-Tabelle verwendet werden, um die Abstandsinformationen einer bestimmten Gewebeart zuordnen zu können. Als Funktion von Dichte $\rho$ und Kompressionsmodul K beträgt die Phasengeschwindigkeit $c = K/\rho$. Dabei ist der Kompressionsmodul K definiert über $\Delta p = -K \, \Delta V/V$, d.h. als reziproke Kompressibilität. Als Funktion der Kompressibilität ist die Schallgeschwindigkeit somit $V_{FI} = sqrt(1 / \rho \beta_{iso})$. Hier steht ISO

für die isotherme Kompressibilität. Die lokale Geschwindigkeit von Teilchen bezieht sich ebenfalls auf eine Welle, deren Amplitude als Schallschnelle $v_0$ bezeichnet wird. Sie kann auch als Funktion der Druckamplitude beschrieben sein und beträgt dann $v_0 = c \cdot \rho_0$, mit $\rho_0$ als relative Dichteschwankung. Die Geschwindigkeit der Teilchen im akustischen Feld ist proportional zur relativen Dichteschwankung und damit auch zur Druckänderung. Die Schallimpedanz, stellt das Verhältnis von Schalldruck zu Schallschnelle dar. Die Schallimpedanz spielt beim Ultraschall eine ähnliche Rolle wie die Impedanz in der Elektronik. In Flüssigkeiten und Gasen ist die Impedanz Z in guter Näherung reell, die Druckänderung und die Schallschnelle laufen also in Phase und die Absorption ist gering. Die Intensität einer Schallwelle, d.h. der Energietransport pro Fläche und Zeiteinheit, ist j = Kraft ·Weg / Fläche . Zeit = p · c. Bei der Berechnung der Abstandsinformationen können Reflexionswerte vorgegeben sein und mit den gemessenen Werten (Soll/Ist-Vergleich) verglichen werden. Hierauf können Warnsignale ausgegeben werden. Außerdem kann der Motor entsprechend eingeschaltet bleiben oder ausgeschaltet werden. Parallel kann dies auf einem Monitor dargestellt werden.

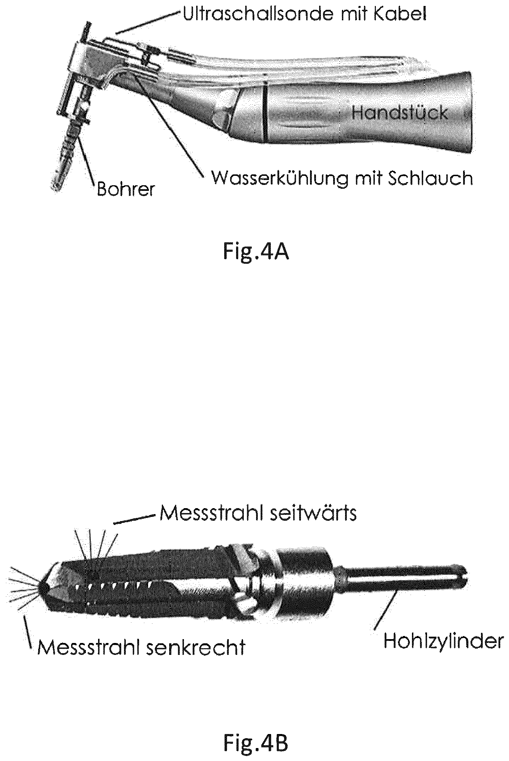

Figur 4A zeigt eine schematische Darstellung eines Bohrers mit Bohrer, Bohrkopf, und Sensoreinheit und stellt eine bildliche Teildarstellung des medizinischen Bohrsystems aus Figur 1 dar. Hier kann die Sensoreinheit ein oder mehrere Schallwandler, oder ein optischer Interferometer sein. Die Sensoreinheit kann zu dem in dem Bohrkopf angeordnet sein oder sich zumindest Weise darin befinden. Um die Hitze beim Bohren abzutransportieren wird hier schematisch eine Wasserkühlung dargestellt. Die Sensoreinheit wird schematisch in Figur 4A als Schallwandler dargestellt. Die Sensoreinheit kann auch als optisches Interferometer ausgebildet sein über optische Kohärenztomographie Daten zu erfassen. Die Sensoreinheit kann zum Beispiel in einem axialen Schacht angebracht sein, siehe Figur 4A. Die Sensoreinheit kann ferner auf einem Kopf eines Winkelstücks des Bohrers angebracht sein. Wenn die Sensoreinheit auf dem Kopf des Winkelstücks angebracht ist, kann die Verarbeitungseinheit dazu ausgebildet sein, eine längere Meßtiefe aufgrund der Bohrerlänge heraus zu rechnen. Wenn die Sensoreinheit in dem Bohrkopf angebracht ist, kann die Verarbeitungseinheit dazu ausgebildet sein, die längere Meßtiefe nicht heraus zu rechnen. Im Folgenden wird eine Anordnung der Meßstrahlen, die zur Laufzeitmessung verwendet wird, dargestellt.

Figur 4B zeigt eine schematische Darstellung eines Bohrkopfs und Meßstrahlen. Der Bohrkopf kann so beschaffen sein, dass die Sensoreinheit darin integriert ist. Ferner kann der Bohrer als Anpassungsstück für einen zur Speisung von Meßstrahlen verwendeten Hohlzylinder vorgesehen sein. Die Meßstrahlen können einerseits senkrecht und andererseits seitwärts zur Vorrichtung ausgesendet werden. Ferner kann vorgesehen sein, dass zum einen die Meßstrahlen senkrecht abgestrahlt werden können und aus dieser Richtung genauso empfangen werden können. Das gleiche gilt für den seitwärts gerichteten Meßstrahl. Der empfangene Meßstrahl ist das Echo von dem gesendeten Meßstrahl, wobei sich das Echo von dem gesendeten Meßstrahl aufgrund von Dämpfung und Reflexion in seiner Amplitude unterscheidet. Ferner kann es zu einer frequenzmäßigen Veränderung des Echos kommen. Die frequenzmäßige Veränderung ist in Figur 5B gezeigt. Beispiele für empfangene Echos sind in Figur 5A gezeigt.

Figur 5A zeigt eine schematische Darstellung eines Reflexionen Diagramms anzeigend verschiedene Laufzeitmessungen. Hier sind Echos bezüglich der Bohrertiefe, der Distanz zu Nerven und zusätzliche Spätechos (Tiefenskala) dargestellt. Die Spätechos sind charakteristisch für Schleimhautschwellungen, Sekretverhaltungen, Zysten oder neoplastische Veränderungen. Figur 5A bezieht sich beispielhaft auf Ultraschallimpulse, die durch eine Kieferhöhle bzw. Stirnhöhle gesendet werden. Die verwendete Schallsonde kann dabei gleichzeitig als Sender und Empfänger von Echos dienen, deren Eigenschaft von den, zum Beispiel Nasennebenhöhlen, angetroffenen Strukturen abhängt. Die Echos bilden sich bei einer Änderung der durch den Meßstrahl getroffenen Impedanz. Zum Beispiel an den Grenzflächen zwischen Knochen und Gewebe, Knochen und Flüssigkeit sowie zwischen allen festen und flüssigen Materialien und Luft. Die einfache und schnell durchzuführende Ultraschalluntersuchung ist eine unschädliche Untersuchungsmethode zur Visualisierung von zum Beispiel Maxilares und Frontalis. Diese dient zum Ersatz/Ergänzung der Röntgendiagnostik, insbesondere bei Kindern, Schwangeren und Verlaufskontrollen bei Sinusitiden. Bei gewöhnlichen Ultraschalluntersuchungen wird ein Kopplungsgel verwendet, das einen Impedanzunterschied zwischen Haut und Luft verringert, somit kommt es an dieser Stelle zu weniger Reflexionen, folglich zu weniger Echos an dieser Stelle. Folglich wird die Genauigkeit der Messung des zu untersuchenden Bereichs verbessert. Das reflektierte Echo kann sich nach dem Reflexionsgesetz

$$R = \left( \frac{z_1 - z_2}{z_1 + z_2} \right)^2$$

ergeben, wobei R = Reflexionsvermögen, $Z_1$ = erste Impedanz an einer Grenzfläche, und $Z_2$ = zweite Impedanz an derselben Grenzfläche. Dieser Effekt wird zur Berechnung der Laufzeit des gesendeten Meßstrahls verwendet. Die Laufzeit kann sich dadurch ergeben, dass der Meßstrahl eine Strecke zurücklegt und an einer Grenzfläche reflektiert wird, wobei der reflektierte Meßstrahl von der Ultraschallsonde empfangen wird. Der reflektierte Meßstrahl kann somit ein Teil des gesendeten Meßstrahls sein, zumindest in einer gedämpften und umgelenkten Form. Eine weitere zu verwerten Information kann die Frequenz sein. Das wird in Figur 5B schematisch dargestellt.

Figur 5B zeigt eine schematische Darstellung eines Puls-Doppler Verfahrens. Eine Sensoreinheit, hier beispielhaft der Wandler d, sendet ein Signal in Richtung einer hier schematisch als relativ groß dargestellte Ader. In einer relativ großen Ader wird ein parabolisches Geschwindigkeitsprofil erwartet. Eine Dopplermessung führt in diesem Fall zu einer Verteidigung weiter bei diesem Fall zu einer Verteilung von Frequenzverschiebungen von Null (am Rand) bis zu einem Maximum (im Zentrum des Gefäßes). Eine Messung kann im Zeitbereich, bei der über die Ader gemittelt wird, eine Fouriertransformierte von ausgesendeten Meßstrahlen ergeben. Bei einer homogenen Geschwindigkeitsverteilung ist die Frequenz im gesamten Gefäß um den gleichen Betrag verschoben. Das kann zum Finden von Einengungen oder Turbulenzen führen. Da sich feste Körperteile, so wie Knochen, nicht bewegen, kann somit eine Gewebeart durch das Verfahren entsprechend Figur 2 entdeckt werden, wenn das Puls-Doppler Verfahren zur Entdeckung von nicht zu bohrenden Gewebearten angewandt wird. Dabei kann eine amplitudenbasierte und frequenzbasierte Berechnung in der Verarbeitungseinheit, wie in Figur 1 gezeigt, angewandt werden. Die Verarbeitungseinheit kann ferner dazu ausgebildet sein, die Frequenz der gesendeten und der empfangenen Meßstrahlen zu vergleichen und/oder die Amplitude der gesendeten und der empfangenen Meßstrahlen zu vergleichen. Hierfür kann ein Vergleich im Frequenz- und/oder im Zeitbereich vorgesehen sein. Die Verarbeitungseinheit kann ferner dazu vorgesehen sein, die Steuereinheit mit Informationen über den Vergleich zu versorgen. Die Verarbeitungseinheit kann dazu ausbildet sein, eine frequenzmäßige Verschiebung zwischen den gesendeten und den empfangenen Meßstrahlen zu bestimmen. Die Verarbeitungseinheit kann ferner dazu ausgebildet sein, basierend auf der frequenzmäßigen Verschiebung, der Steuereinheit mitzuteilen, dass es sich um eine nicht zu bohrende Gewebeart handelt. Daraufhin kann die Steuereinheit ein Signal basierend auf der Mitteilung auslösen. Dies kann auch ein Stoppen des Bohrers umfassen.

Die Erfindung ist natürlich nicht in irgendeiner Weise auf die zuvor beschriebenen Ausführungsformen beschränkt. Es werden im Gegenteil viele Möglichkeiten für Modifikationen daran einem Durchschnittsfachmann ersichtlich, ohne von der zugrundeliegenden Idee der Erfindung abzuweichen, wie sie in den beigefügten Ansprüchen definiert ist.

**Patentansprüche**

1. Verfahren zum Steuern eines medizinischen Bohrsystems, das Verfahren umfassend:

kontinuierliches Aussenden von Meßstrahlen;
kontinuierliches Empfangen der teilweise an verschiedenen Gewebearten reflektierten Meßstrahlen;
Verarbeiten der kontinuierlich empfangenen Meßstrahlen zu Abstandsinformationen, wobei die Abstandsinformationen Abstände zwischen einem Bohrkopf des Bohrsystems und den verschiedenen Gewebearten angeben;

Auswerten, basierend auf den Abstandsinformationen, ob es erlaubt ist, in Richtung einer Bohrung des Bohrkopfs zu bohren; und Steuern des Bohrkopfs basierend auf dem Auswerten.

2. Verfahren nach Anspruch 1, ferner umfassend:

Anzeigen von zu bohrenden und nicht zu bohrenden Gewebearten basierend auf dem Auswerten.

3. Verfahren nach Anspruch 1 oder 2, wobei das Anzeigen ferner Anzeigen von unterschiedlichen Gefahrenstufen der nicht zu bohrenden Gewebearten und/oder Vorwarnungen für in Richtung der Bohrung liegende nicht zu bohrende Gewebearten und/oder Vorwarnungen für in angrenzender Nähe der Bohrung liegende nicht zu bohrende Gewebearten umfasst.

4. Verfahren nach einem der vorangegangenen Ansprüche, ferner umfassend:

Auslösen eines Signals, wenn das Auswerten ergibt, dass es nicht erlaubt ist, mindestens eine der verschiedenen Gewebearten in Richtung der Bohrung zu bohren.

5. Verfahren nach einem der vorangegangenen Ansprüche, wobei das Steuern umfasst:

Stoppen der Bohrung vor Eindringen des Bohrkopfs in mindestens eine der nicht zu bohrenden Gewebearten.

6. Verfahren nach einem der vorangegangenen Ansprüche, wobei die Abstandsinformationen basierend auf einem Dämpfungsgesetz berechnet werden, und wobei charakteristische Frequenzen für zu bohrende Gewebearten und nicht zu bohrende Gewebearten für das Berechnen verwendet werden.

7. Computerprogramm zur Durchführung eines Verfahrens nach einem der vorangegangenen Ansprüche.

8. Computerprogrammprodukt beinhaltend einen maschinenlesbaren Programmträger, auf dem ein Computer-Programm gemäß dem vorherigen Anspruch in der Form von elektronisch und/oder optisch lesbaren Steuersignalen gespeichert ist.

9. Medizinisches Bohrsystem umfassend:

einen Bohrer, der konfiguriert ist, verschiedene Gewebearten zu bohren, wobei der Bohrer einen Bohrkopf umfasst;

eine Sensoreinheit, der konfiguriert ist, Meßstrahlen kontinuierlich auszusenden, und die teilweise an verschiedenen Gewebearten reflektierten Meßstrahlen kontinuierlich zu empfangen;

eine Verarbeitungseinheit, die konfiguriert ist, die kontinuierlich empfangenen Meßstrahlen zu Abstandsinformationen zu verarbeiten, wobei die Abstandsinformationen Abstände zwischen dem Bohrkopf und den verschiedenen Gewebearten angeben, und basierend auf den Abstandsinformationen auszuwerten, ob es erlaubt ist, in Richtung einer Bohrung des Bohrkopfs zu bohren; und

eine Steuereinheit, die konfiguriert ist, den Bohrkopf basierend auf dem Auswerten zu steuern.

10. Medizinisches Bohrsystem nach Anspruch 9, wobei sich die Sensoreinheit zumindest teilweise in dem Bohrkopf befindet, und die Sensoreinheit eine Abstrahlcharakteristik beziehungsweise Empfangscharakteristik aufweist, die in Richtung einer Bohrung und/oder senkrecht dazu weist.

```
                    ┌─────────────────┐
                    │  Referenzdaten  │
                    │                 │
                    └────────┬────────┘
                             │
┌──────────────────┐        │              ┌──────────────────────┐
│  Ultraschalldaten│        ▼              │ Zustandsdaten (Ampel:│
│ (Schallwandler-  ├──┐ ┌───────────┐  ┌──▶│  rot, gelb, grün;    │
│     daten)       │  └▶│           │  │   │ akustisches Signal;  │
└──────────────────┘    │           │  │   │        etc.)         │
                        │           ├──┤   └──────────────────────┘
┌──────────────────┐    │Steuer-    │  │   ┌──────────────────────┐
│ Bohrsteuerung(An/│◀───┤einheit    │  └──▶│  Darstellungsdaten   │
│ Aus, Umdrehungen/│    │           │      │        (OCT)         │
│     Minute )     │    │           │      └──────────────────────┘
└──────────────────┘    └─────▲─────┘
                              │          ┌──────────────────────┐
┌──────────────────┐         │       └─▶│  Überwachungsdaten   │
│     Andere       │◀───┐    │           │  (Tiefe, Entfernung) │
│   Steuersignale  │    │    │           └──────────────────────┘
│   (Vibration)    │    │ ┌──┴──────────┐
└──────────────────┘    │ │ Topologische│
                        │ │ Daten       │
                        │ │(aktuell     │
                        │ │ erwartetes  │
                        │ │ Material)   │
                        │ └─────────────┘
```

# Fig.1

Fig.2

Dämpfung im Gewebe

| Absorptions-koeffizient $\alpha$ | | Dämpfungsgesetz: $p(z) = p_0 \cdot e^{-\alpha \cdot f \cdot z}$ |
| Eindringtiefe z | | p(z): Schalldruck<br>$p_{(0)}$ : Anfangsschalldruck<br>$\alpha$ : Absorptionskoeffizient<br>z : Eindringtiefe<br>f : Frequenz |
| Frequenz f | | |

Fig.3A

Eindringtiefe – Untersuchungsgebiet

| Frequenz f | Eindringtiefe z | Untersuchungs-gebiet |
|---|---|---|
| 1,0 MHz | 50 cm | Fetus / Leber, Herz |
| 3,5 MHz | 15 cm | Niere |
| 5,0 MHz | 10 cm | Gehirn |
| 7,5 MHz | 7 cm | |
| 10,0 MHz | 5 cm | Prostata (endo) / Pankreas (intraoperativ) |
| 20,0 MHz | 1,2 cm | Auge, Haut |
| 40,0 MHz | 0,60 cm | Haut, Gefäße |

Fig.3B

Fig.3C

Akustische Impedanzen

| Medium | Impedanz $(10^6 \cdot Ns/m^3)$ |
|---|---|
| Lebergewebe | 1,59 |
| Wasser | 1,49 |
| Luft | 0,00041 |
| Knochen | 3,75–7,8 |

Fig.3D

| Gewebe | Dämpfung in dB/cm |
|---|---|
| Blut | 0,18 |
| Fett | 0,6 |
| Niere | 1,0 |
| Muskel$_{\text{in Faserrichtung}}$ | 1,2 |
| Muskel$_{\text{quer z. Faserrichtung}}$ | 3,3 |
| Gehirn | 0,85 |
| Leber | 0,9 |
| Lunge | 40,0 |
| Knochen | 20,0 |

Fig.3E

Ultraschallsonde mit Kabel

Handstück

Bohrer

Wasserkühlung mit Schlauch

Fig.4A

Messstrahl seitwärts

Messstrahl senkrecht

Hohlzylinder

Fig.4B

Fig.5A

Fig.5B